# EUROPÄISCHE PATENTANMELDUNG

(11) **EP 4 789 675 A1**
(43) Veröffentlichungstag der Anmeldung: **12.08.2026**
(21) Anmeldenummer: 25156883.8
(22) Anmeldetag: 10.02.2025
(51) Int. Cl.: A61K 35/646, A61K 35/00, A61K 35/56, A61K 36/185, A61P 15/00, A61P 15/08, A61P 15/10

(54) **ZUSAMMENSETZUNG ZUR BEHEBUNG EREKTILER DYSFUNKTION**

(71) Anmelder: Patentpool Target GmbH, 80331 München (DE)
(72) Erfinder: Burda, Renate, 82054 Sauerlach (DE); Sand, Elisabeth, 91567 Rauenzell/Herrieden (DE); Mörth-Kretschmer, Jenny, 21244 Buchholz in der Nordheide (DE); Weickmann, Dirk, 91567 Rauenzell/Herrieden (DE)
(74) Vertreter: Reich, Jochen

(57) **Zusammenfassung**

Vorgeschlagen wird eine pharmazeutische Zusammensetzung zur Behandlung der erektilen Dysfunktion, enthaltend in einer pharmazeutisch wirksamen Menge Wirkstoffe aus dem Gesamt-Giftcocktail von Spinnen der Gattungen Phoneutria, Viridasius und/ oder Ancylometes und/ oder dem Extrakt von jungen Blättern von Urtica bianorii, sowie eine für den jeweiligen Verwendungszweck abgestimmte Menge an Salbengrundlage.

## Beschreibung

Vorgeschlagen wird eine pharmazeutische Zusammensetzung zur Behandlung der erektilen Dysfunktion, enthaltend in einer pharmazeutisch wirksamen Menge Wirkstoffe aus dem GesamtGiftcocktail von Spinnen der Gattungen Phoneutria, Viridasius und/ oder Ancylometes und/ oder dem Extrakt von jungen Blättern von Urtica bianorii, sowie eine für den jeweiligen Verwendungszweck abgestimmte Menge an Salbengrundlage. Es wurden Wirkstoffe aus den Gesamtgiftcocktails von Spinnenarten aus den Gattungen Ancylometes, Phoneutria und Viridasius und den Blättern von Urtica bianorii isoliert, die bei äußerlicher Anwendung mittels Salbe therapeutisch positive Effekte bei der Behandlung aus dem Formenkreis erektiler Dysfunktionen aufweisen. Die äußerliche Behandlung zur Behebung von Erektionsstörungen unter Ausschluss von auch systemischen Erkrankungen mit ggf. auch traumatisch bedingter Vorschädigung als ursächlichen Hintergrund ist dabei weitestgehend nebenwirkungsfrei.

Auf der Suche nach neuen Wirkstoffen für die Behandlung von Krankheiten oder Störungen des menschlichen Organismus wir von vielen privaten und staatlichen Forschungsinstituten zunehmend im Bereich der in der Natur vorkommenden, natürlichen chemischen Verbindungen geforscht. Zu erwähnen sind hier die biogenen Gifte und Mund- oder Hautsekrete unterschiedlicher Tierfamilien. Ebenso aber auch die mittlerweile in Pflanzen nachgewiesenen Substanzen ähnlicher Wirkmechanismen.

Die Nutzung biogener Gifte begann in der Geschichte der Menschheit schon in der Urzeit als sie zur Verlegung von Beutetieren mit vergifteten Waffen diente. Zur gefahrlosen Anwendung dieser Gifte waren jedoch von Anfang an gewisse Grundkenntnisse über deren Behandlung und Wirksamkeit erforderlich. Die weiter durchgeführten Versuche, die Zusammensetzung des chemischen Aufbaus biogenen Gifte zu entschlüsseln, führen später zur gezielten Suche bestimmter Wirkstoffe als eigentliche Verursacher beobachteter Wirkung.

Insbesondere nach der von Paracelsus (1483-1514) erhobenen Forderung, die Wirkstoffe von Arzneipflanzen zu isolieren, die zur Entwicklung der latrochemie, als der Chemie hinsichtlich ihres ärztlichen Anwendungsbereichs beitrug, dürften diese Bemühungen verstärkt haben. Vor allem die Kunst des Destillierens von Stoffen wurde in den Dienst der Forschung gestellt und lieferte eine Vielzahl ätherischer Öle und flüchtiger Stoffe. Aber für die Isolierung anderer Wirkstoffe oder gar für deren chemische Aufschlüsselung waren die damals bekannten Methoden unzureichend. Erst zu Beginn des 19.Jahrhunderts war die Entwicklung der technischen Fertigkeiten in der Chemie weit genug fortgeschritten, die Ära der Isolierung von reinen Wirkstoffen aus biologischem Material einzuleiten.

Zunächst nutzte man zur Abtrennung der gesuchten Wirkstoffe von den Begleitstoffen, die Unterschiede in der Löslichkeit der untersuchten Substanzen in verschiedenen Lösungsmitteln. Beobachtet wurden hierbei zum Beispiel mit Fällungsmitteln die Unterschiede im Verteilungsverhalten zwischen zwei nicht mischbaren flüssigen Phasen in der Flüchtigkeit und der chemischen Reaktivität. Einen gewaltigen Aufschwung in der Trenntechnik, dem Weg zur Ermittlung von Wirkstoffen zur Bekämpfung von Krankheiten machte die Entwicklung chromatographischer Verfahren in der Mitte des 20. Jahrhunderts möglich.

Ausgehend von der Verteilung zwischen einer mobilen und einer stationären flüssigen Phase, von der Adsorption, den Molekülsiebeffekten, dem lonenausstausch, der Affinität (insbesondere von Proteinen) zu bestimmten chemischen Verbindungen (z.B. Enzymsubstraten) und der Beweglichkeit geladener Moleküle im elektrischen Feld, wurde eine Vielzahl neuer Trenntechniken entwickelt.

Derzeit werden z.B. Tumore als die gefährlichsten und gefürchtetsten Krankheiten unserer Zeit immer noch auf eine sehr radikale und wenig umweltschonende Weise bekämpft, obwohl natürliche aus Tiergiften gewonnene Wirkstoffe auch eine echte Krebstherapie einleiten könnten. Ähnliche Ansätze sind mittlerweile auch mit Pflanzenwirkstoffen denkbar.

Im Gegensatz zur sogenannten Schulmedizin wurde aber auch hier immer wieder versucht eine Krebstherapie, die ihren Namen verdient, auf subtile Weise zu ermöglichen. Zu diesem Zweck wurde auf den reichen Schatz der Natur zurückgegriffen.

Es werden hierzu, unter anderem, viele aus giftigen Lebewesen isolierte, stark wirksame Stoffe in therapeutischen Dosen als Arzneistoffe genutzt.

So ist aus der DE 19961141 A1 ein pharmazeutischer Wirkstoff bekannt, bei dem gefunden wurde, dass Bestandteile der Spinnengifte von Spinnen der Familie Sicariidae zur Behandlung von Tumorerkrankungen verwendet werden können. Es werden hierbei in der Hauptsache Peptidtoxin aus dem Gift dieser Spinnenarten, eine weitere aus dem Gift gewonnene antagonistisch wirkende Substanz und/oder eine Kombination dieser Bestandteile genutzt.

In der Folgezeit wurden zahlreiche Wirkstoffe gegen verschiedene Tumorarten und andere Krankheiten oder Dysfunktionen auf der Basis biogenen Gifte und anderer biogener Wirkstoffe entwickelt, welche sich auch als geeignet erwiesen haben auf natürliche Weise eine erektile Dysfunktion erfolgreich und bedarfsgerecht zu kompensieren.

Neuerer Stand der Technik zur Behebung erektiler Dysfunktionen sind derzeit verschreibungspflichtige Medikamente nach dem biochemischen Prinzip PDE-5-Inhibitoren. Diese Medikamente enthalten als Wirkstoffe Sildenafil.

All diese Medikamente wirken nicht automatisch. Eine Erektion erfolgt vielmehr nur bei sexueller Stimulierung, welche mechanisch und/oder psychisch erfolgen kann.

Verschiedene Pressemeldungen über den beabsichtigten Einsatz von dem Heilkraut "Elfenblume" basieren auf Berichten aus der traditionellen chinesischen Heilmedizin und befinden sich noch ausschließlich im Experimentierstadium bzw. haben keine Marktreife.

Weitgehend nebenwirkungsfrei, auf der Basis von Wirkstoffen aus Spinnengiften wirkende und zur äußerlichen Anwendung bestimmte Salben zur zeitlichen Behebung erektiler Dysfunktionen, gibt es derzeit (mit Ausnahme der gegenständlichen Patentanmeldung) nicht.

Den Anspruch auf weitestgehende Nebenwirkungsfreiheit verbunden mit der entwickelten Anwendungsform erhebt derzeit nur die gegenständliche Patentanmeldung nach dem uns bekannten Stand der Forschung. Durch die nur lokale Applikation der natürlichen Wirkstoffe auf die Haut der primären Erfolgsorgane (Penis und Hoden) als unmittelbarer Zielort einer Wirkungsentfaltung wird von vornherein die Nebenwirkungen begünstigende Belastung des gesamten Körpers in einer dadurch zwangsläufig höheren Dosierung der beim oralen Einsatz hauptgebräuchlichen synthetischen Wirkstoffe wie den PDE-5 Hemmern, Sildenafil, Tardalafil und ggf. anderen, die z.B. mit einer zusätzlichen Belastung des Herz-Kreislauf-Systems verbunden sind, vermieden.

Unsere bisherigen Forschungsarbeiten haben Meldungen anderer Forscher dahingehend widerlegt, dass nur der Biss der Kammspinne Phoneutria nigriventer eine langanhaltende Erektion des Penis auslöst, auch aus der einschlägigen Literatur hinlänglich bekannt ist (z.B. Mebs D. (2000); Gifttiere: Ein Handbuch für Biologen, Toxokologen, Ärzte und Apotheker, 2.Aufl., Wissenschaftliche Verlagsgesellschaft Stuttgart).

Wir haben herausgefunden, dass auch bei anderen Angehörigen der Familie Ctenidae wie der Gattung Ancylometes (Betrau, 1880) und Spinnen der Gattung Viridasius (Lenz, 1886) aber auch bei einer der von uns untersuchten Urticaceae bei Gebissenen eine Erektion des Penis ausgelöst worden ist.

Es ist Aufgabe der erfindungsgemäßen pharmazeutischen Zusammensetzung, Mittel und Verfahren zur Behandlung der erektilen Dysfunktion zur Verfügung zu stellen. Zudem soll die erfindungsgemäße Zusammensetzung als "Salbenwirkstoff" zur äußerlichen Behandlung einsetzbar sein. Das Problem aller bisher bekannten und auf dem Markt befindlichen Erektionshilfsmittel welcher in Form von Tabletten oder Säften eingenommen werden müsse, liegt in der mehr oder minder starken Belastung von Herz und Kreislauf, anderen organbezogenen Nebenwirkungen und Beeinträchtigungen des Wohlbefindens wie Kopfschmerzen, Sehstörungen, Überreizung des Magens oder Irritationen der Nasenschleimhaut.

Zum Pflichtenheft der neuen Erfindung- Salbe zur Behebung erektiler Dysfunktionen- gehört deshalb die Vermeidung aller Nebenwirkungen der bisher zur Verfügung stehenden und oral einzunehmender Mittel zur Behebung erektiler Dysfunktionen. Diese Aufgabe wird von Wirkstoffen mit geeigneten Molekulargewichten, als Bestandteil des Giftes bestimmter Jagdspinnen gelöst, wobei die neue Systematik der Gattung Phoneutria maßgeblich ist und dieser Patentschrift zugrunde liegt. Lit.: Elksnat B. (2000).

Die Forschungsergebnisse-Salbe zur Behebung erektiler Dysfunktionen- erheben den Anspruch, durch eine gezielte Auswahl und eine noch differenziertere Gewinnung, von Spinnengiften ein zuverlässiges und nebenwirkungsarmes Hilfsmittel zur Überwindung erektiler Dysfunktionen herzustellen.

Durch gezielten und genau dosierten Einsatz von speziellen, extrahierten Wirkstoffen ausgewählter und mit Spezialmethoden gewonnener Spinnengifte der Gattungen Phoneutria, Viridasius und Ancylometes, sowie Extrakten von Urtica bianorii, integriert in eine Trägersubstanz in Salbenform können zu jedem vom Anwender bestimmbaren Zeitpunkt mit vernachlässigter Vorlaufzeit nicht ausschließlich organisch krankheitsbedingte Erektionsstörungen/ Erektionsschwächen des männlichen Penis situationsbezogen und bei gleichzeitiger Steigerung des Lustgefühls für die jeweils relevante Zeitspanne behoben werden.

Es handelt sich gemäß einem Aspekt der vorliegenden Erfindung um eine Salbe mit tierischen und/ oder pflanzlichen Wirkstoffen zur Behebung der erektilen Dysfunktion.

Die Anwendung der Salbe erfolgt beim geschlechtsreifen Mann rein äußerlich im Bereich der Peniswurzel und der Hoden und ist frei von den bei herkömmlichen, medizinischen Heilmitteln in unterschiedlicher Form auftretenden Nebenwirkungen/Nebenfolgen/Gefahren.

Die Erfindung - Salbe zur Behebung erektiler Dysfunktionen - erhebt den Anspruch auf der Basis ausschließlich natürlicher Wirkstoffe, gewonnen aus den Gesamtgiftcocktails von Jagdspinnen der Gattungen Ancylometes, Phoneutria und/oder Viridasius (siehe Tabelle 1 und 2), sowie Extrakten aus den Blättern von Urtica bianorii, Erektionsstörungen des menschlichen Penis, verursacht durch psychosomatische Störungen, Störungen der Durchblutung, hormonelle Störungen und ähnliche, ggf. auch bisher noch unbekannten Störungen, situationsbezogen und zeitlich beschränkt nach den Wünschen, Bedürfnissen und Befindlichkeiten des Anwenders zu beheben, ohne dabei einen medikamentösen Eingriff vorzunehmen und sich den Gefahren der bereits bekannten bzw. möglichen Nebenwirkungen der herkömmlichen medikamentösen Anwendung auszusetzen. Die Erfindung erhebt hierbei den Anspruch Nebenwirkungsarm, bzw. Nebenwirkungsfrei zu sein.

Weitere Vorteile der Erfindung-Salbe zur Behebung erektiler Dysfunktionen- sind:
- Gezielte und substanzneutrale Anwendungsmöglichkeiten
- Keine Gewöhnung an die in der Salbe enthaltenen Wirkstoffe
- Gesteigertes, natürliches Lustempfinden
- Gesteigertes Selbstwertgefühl
- Abbau von Angstgefühlen
- Bestätigung, dass eine vermutete bzw. befürchtete, gravierende organische Vorschädigung als Ursache der erteilen Dysfunktion weitgehend auszuschließen ist.

Mit der vorgeschlagenen Erfindung ist es wissenschaftlich nachgewiesen gelungen,
a) ein allen Tierschutz-/Arbeitsschutzvorschriften und dem Verwendungszweck gerecht werdendes "Melkverfahren" zur Gewinnung der Tiergifte zu entwickeln und/oder zu optimieren.
b) die für den Patentgegenstand brauchbaren Bestandteile der jeweiligen Tiergifte und Pflanzengifte mit erektionsauslösender Wirkung physikalisch- und/oder labortechnisch zu bestimmen.
c) die für den Patentgegenstand brauchbaren Bestandteile der jeweiligen Tiergifte und Pflanzengifte zu extrahieren und/oder zu kombinieren.
d) die für eine zuverlässige und nebenwirkungsfreie, zeitweise Beseitigung, erektiler Dysfunktionen erforderliche Gesamtdosis zu bestimmen und/oder zu erproben.
e) eine geeignete Trägersalbe zur problemlosen und/oder nebenwirkungsfreien Anwendung zu finden und/oder auszuwählen und/oder zu erforschen.

Der Wirkstoff wird und/ oder die Wirkstoffe werden aus dem Gesamtgiftcocktail der Spinnen und/ oder der Mallorca-Brennnessel (Mallorquinische Brennnessel) gewonnen. Die Spinnen werden dazu gemolken. Von der Brennnessel werden die Blätter wässrig oder alkoholisch extrahiert. Das Melkverfahren ist wie nachstehend beschrieben durchzuführen. Zunächst werden die Spinnen ruhiggestellt. Dafür werden die Spinnen samt Behältnis in einen Kühlschrank gestellt, der eine Temperatur von 7-17 Grad Celsius, bevorzugt 10-15 Grad Celsius, aufweist. Im Kühlschrank werden die Spinnen nach 15-30 Minuten Kältegelähmt und können dann gefahrlos aus dem Behältnis genommen werden. Die Spinnen werden von Rechtshändern mit der linken Hand aus dem Behältnis genommen, von den Linkshändern mit der rechten Hand aus dem Behältnis genommen.

Die Spinnen werden mit der Bauchseite nach oben gedreht, während man sie hält. Man lässt die Spinnen bei einer bevorzugten Temperatur von 20-23 Grad Celsius in ein steriles Wattestäbchen beißen, wobei eine Temperatur von 22 Grad Celsius besonders bevorzugt ist.

Man gibt die Spinnen zurück ins Behältnis.

Das "Melken" soll gemäß einem Aspekt der vorliegenden Erfindung 5 Minuten pro Spinne nicht überschreiten.

Die Pflanzen werden für die erfindungsgemäße Verwendung wie folgt extrahiert:
Die erfindungsgemäße Zusammensetzung wird auf die folgende Weise hergestellt:
a) Es wird eine bestimmte Menge Blätter, bevorzugt frische Blätter aus der Blattknospe über dem Rhizom in eine bestimmte Menge 96% Ethanol, bzw. bevorzugt 100% Ethanol oder wahlweise in eine bestimmte Menge Aqua ad injectabilia eingebracht.
b) Der Extrakt aus a) wird mindestens 24 Stunden bei Temperaturen von bevorzugt 25 Grad Celsius stehen gelassen. Anschließend werden die Blätter abfiltriert.
c) Dieser Wirkextrakt aus b) wird in 150 ml verflüssigten Bioblütenhonig unter ständigem Rühren eingearbeitet. Wahlweise kann auch der pure Extrakt verwendet werden.

Die Gewinnung bzw. Herstellung der für die Salbe erforderlichen Wirkstoffe hinsichtlich Zusammensetzung, Wirksamkeit, wissenschaftlicher Bestimmbarkeit und der erforderlichen Reinheit basiert auf der vom Erfinder der vorliegenden Anmeldung entwickelten und patentrechtlich geschützten Methode des Auftrennens der Giftcocktails über die Säulen-Chromatographie.

Säulen-Chromatographie ist ein chromatographisches Trennverfahren, bei dem eine flüssige oder gelöste Probe durch eine mit einem geeigneten Trägermaterial (z. B. Gel, Silica oder Harz) gefüllte Säule geleitet wird. Die unterschiedlichen Wechselwirkungen der Komponenten mit dem Trägermaterial führen zu einer unterschiedlichen Verweilzeit, wodurch die Bestandteile der Probe separiert und anschließend fraktioniert werden können.

Bei dem Verfahren zur Herstellung einer pharmazeutischen Zusammensetzung wurde herausgefunden, dass der biogene Wirkstoff (Gesamtgift) der jeweilig angegebenen Gattung bzw. Art:
- eingeengt werden kann,
- Auf minus 20 Grad Celsius, gefroren werden kann,
- In ein Vakuum verbracht und auf plus 4 Grad Celsius, sublimiert werden kann, um einem späteren Zeitpunkt in eine Salben- oder Cremeträgersubstanz eingebracht zu werden.

Die gegenständliche Erfindung-Salbe zur Behebung erektiler Dysfunktionen- wird bei gewünschtem Bedarf und situationsbezogen im Bereich der Peniswurzel und der Hoden aufgetragen und massiert, wobei ca. 2 Fingerspitzen / etwa 2ml) genügen, ohne die Möglichkeit einer schädlichen Überdosierung. Die erforderliche Dosis wurde von den Patienten (siehe " Patientenbeispiele") mit ihren jeweiligen Therapeuten/Therapeutinnen empirisch bestimmt.

Die erfindungsgemäße Salbe löst dann bei bestimmungsgemäßer Anwendung aufgrund seiner enthaltenen Wirkstoffe nach Aussage von Patienten eine bis zu 40 Minuten anhaltende natürliche Erektion des männlichen Gliedes aus, sofern beim Anwender keine schweren organischen Grunderkrankungen, welche bereits ursächlich und mechanisch eine Erektion behindern/verhindern, vorliegen.

Nachdem die erfindungsgemäße Salbe in der Auswahl und der Dosierung der Wirkstoffe so aufgebaut ist, dass Überdosierung und somit praktisch alle denkbaren Anwendungsfehler ausgeschaltet sind, erhebt die Erfindung den Anspruch weitgehend frei von Nebenwirkungen bzw. besonders nebenwirkungsarm zu sein.

Selbst eine versehentliche orale Aufnahme der Salbe ist bedenkenlos, da alle in der Salbe enthaltenen Substanzen und Wirkstoffe ohne Schäden und schädliche Nebenwirkungen verdaut und somit abgebaut und ausgeschieden werden.

Als geeignet gelten aufgrund eigener Forschungen Wirkstoffe der Gifte nachstehender Spinnen mit den entsprechenden Molekulargewichten:

**Tabelle 1**

| | |
|---|---|
| Phoneutria fera | 13,6 kDA |
| Phoneutria depilata | 14,1 kDA |
| Phoneutria boliviensis | 16,2 kDA |
| Phoneutria eickstedtae | 15,4 kDA |
| Phoneutria pertyi | 15,2 kDA |
| Viridasius cf. fasciatus "forestform" | 63,2 kDA |
| Ancylometes bogotensis | 28,2 kDA |
| Ancylometes rufus | 27,6 kDA |
| Urtica bianorii | 15,3 kDA |

Ein Aspekt der erfindungsgemäßen Zusammensetzung besteht darin, dass bei der Untersuchung ungereinigter biogener Wirkstoffe eine erhöhte Wirksamkeit bei männlichen Säugetieren in Bezug auf deren Erektionsfähigkeit bzw. Erektionsgeschehen, gefunden wurde.

Die zur Gewinnung von Spinnengiften erforderliche Melktechnik wird von uns wie nachstehend beschrieben, optimiert und verfeinert.

Bei allen drei Gattungen Phoneutria, Viridasius und Ancylometes handelt es sich um Vertreter der Jagdspinnen, welche sehr schnell und zumeist nicht einfach zu greifen sind. Eine Ruhigstellung der Tiere mittels CO2 oder Schockfrosten lehnen wir aus Tierschutzgründen ab, ist aber erfindungsgemäß möglich.

Trotzdem müssen die Tiere zunächst ruhiggestellt werden. Dazu gibt man sie samt Behältnis für ca. eine halbe Stunde in einen Kühlschrank mit Glastüre und Belüftet bei ca. 10-15 Grad Celsius. Anschließend testet man mit einer langen Pinzette oder einem langen Bambusstecken, ob die Spinne schon ruhiggestellt. Wenn die Spinne ruhig ist, nimmt man sie vorsichtig (bei Rechtshändern) aus dem Haltungs- und Zuchtbehältnis und nimmt sie von oben mit der linken Hand und dreht sie auf den Rücken, so dass die Kiefer nach oben schauen. Dann nimmt man ein steriles Wattestäbchen und "ärgert" die Spinne etwas, so dass sie, während sie aufwacht und wieder warm wird (die optimalen und besonders bevorzugten Temperaturen bei Melken der gegenständlichen Spinnen liegen zwischen 20 und 30 Grad, besonders bevorzugt bei 22 Grad Celsius), in die Watte beißt und dabei Gift abgibt. Anschließend gibt man die Spinne wieder in ihren Behälter zurück, bevor man die Spinne aus der Hand entlässt, fixiert man sie mit der Pinzette, so dass der Melker beim Loslassen nicht gebissen werden kann. Erst nach Entfernung der Hand aus dem Behältnis, wird die Spinne nicht mehr mit der Fixierhilfe festgehalten und die Fixierhilfe aus dem Becken herausgenommen. Das Melken sollte nicht länger als 5 Minuten dauern, um die Spinne nicht übermäßig zu stressen. Dieses mechanische Melken gibt im Gegensatz zum elektrischen Melken Giftcocktails höherer Qualität. Gänzlich abzulehnen ist nicht zuletzt auch aus ethischen Gründen das Töten des Tieres jedoch erfindungsgemäß möglich. Auch ist das Herauspräparieren der Giftdrüsen und das Aufreinigen der daraus gewonnenen Gifte kostenintensiver als die von uns entwickelte Methode.

Die von uns isolierte und in Tabelle 1 aufgeführten Wirkstoffe im Gift der vorgezeichneten Spinnenarten sind grundsätzlich zur zeitweisen Behebung einer erektilen Dysfunktion geeignet.

Maßgebend für eine physikalisch-chemische Beschreibung des jeweils gewonnenen Wirkstoffes ist jedoch, wie in eigenen Untersuchungen festgestellt bzw. herausgefunden wurden, das jeweilige Molekulargewicht der zum Einsatz kommenden Substanzen.

Es wurden bei einem Teil der nachstehend aufgeführten Tiere folgende Molekulargewichte der zur Erektion des männlichen Penis führenden Substanzen gemessen:

**Tabelle 2**

| | |
|---|---|
| Spinnenart | Wirkstoff Molekulargewicht in kDA |
| Phoneutria fera | 13,6 |
| Phoneutria boliviensis | 16,2 |
| Phoneutria eickstedtae | 15,4 |
| Phoneutria pertyi | 15,2 |
| Phoneutria cf. nigriventer | 5,8 |
| Viridasius cf. fasciatus "forestform" | 63,2 |
| Ancylometes bogotensis | 28,2 |
| Ancylometes rufus | 27,6 |

Tabelle 1 und 2 beschreiben auf der linken Seite die Spinnengattung und rechts das Molekulargewicht des Wirkstoffs. Dieser wurde noch nicht näher bezeichnet, lässt sich jedoch anhand dieses Molekulargewichts eindeutig identifizieren. Cf. steht für einen analogen Wirkstoff der noch nicht beschrieben ist, jedoch dadurch eindeutig gekennzeichnet ist, dass er gleiche Wirkung aufweist jedoch durch ein anderes Molekulargewicht, wie angegeben, gekennzeichnet ist.

Erfindungsgemäß wird erkannt, dass jeder der Wirkstoffe einzelne oder in Kombination mit einer Menge der anderen Wirkstoffe eingesetzt werden kann. Die Wirkstoffe können beliebig kombiniert werden und können sich gegenseitig verstärken.

Die Substanz aus Urtica bianorii wird aus jungen Blättern (die frisch geschnitten sind) gewonnen.

Auf molekular-biochemischer Ebene kann aufgezeigt werden, dass sich der in der Presse diskutierte Wirkstoff der Kammspinnenart Phoneutria nigriventer Tx2-6 mit etwa 5 kDA Molekulargewicht deutlich von den von uns gefundenen Substanzen unterscheidet.

Damit ist nachvollziehbar, dass die erfindungsgemäßen Substanzen sich deutlich vom Stand der Technik abheben und auch effektiver einsetzbar sind. Durch die Verwendung als Salbe unterliegen die tierischen Eiweißverbindungen nicht einer Verdauung, wie sie im Magen-Darm-Trakt stattfinden würde.

Die gewonnenen Substanzen wurden getrennt bzw. isoliert, zum Beispiel säulenchromatographisch, wie in der Anmeldung DE 19961141 A1 beschrieben, und stehen anschließend zur Einbringung in die Trägersubstanz (Salbe, Gel, Creme) bereit. Die Fraktion der der jeweiligen Arten, die den erfindungsgemäßen Wirkstoff enthalten, werden bis zur Weiterverarbeitung bei minus 20 Grad Celsius aufbewahrt.

Als Trägersubstanz für die Anwendung der vorstehend beschriebenen Wirkstoffe hat sich am besten (nach bisherigen Erfahrungen) eine Salbengrundlage geeignet, wie auch nachstehende Definition plausibel macht:
Die Salbe (lat. Unguentum, abgekürzt: Ungt.) ist eine halbfeste und homogen aussehende Zubereitung, die zur Anwendung auf der Haut (z.B. als Wundsalbe) oder auf den Schleimhäuten bestimmt ist. Sie dient der lokalen Wirkstoffapplikation oder der Pflege und dem Schutz der Haut oder Schleimhäute. Sie besteht aus einer fettigen Grundlage aus natürlichen oder synthetischen Stoffen und kann ein einphasiges (z.B. Vaseline) oder mehrphasiges (Wasser-in-Öl-Emulsion) System sein. Wirkstoffe oder Arzneistoffe können in der Lösung oder Dispersion eingearbeitet sein. Die Freisetzung von Arzneistoffen aus Salben lässt sich über die Higuchi-Gleichung berechnen. Salben im weiteren Sinn unterscheiden in:
- Salben in engerem Sinn, einphasig
- Cremes
- Gele und/ oder
- Pasten.

Wichtig ist hierbei die Tatsache, dass die Salbengrundlage auch auf Schleimhäute anwendbar ist. Nach der Higuchi-Gleichung errechnet sich eine sehr gute Freisetzung des Wirkstoffes, der in der Lösung aus der Salbe gut durch die Haut diffundieren kann. Versuche zeigten, das Cantharidinanaloge, die einer gesonderten Anmeldung beschrieben werden sollen und hier nicht weiter Gegenstand der Beschreibung sind, die Aufnahme des Wirkstoffes um ein Vielfaches potenzieren können und selbst eine positive temporäre Wirkung hinsichtlich einer Versteifung des männlichen Gliedes in geringem Ausmaß erzielen können. Diese Analoge sollen in Sierra Leone und in unseren europäischen Farmen aus lebenden und selbst gezüchteten Canthariden gewonnen werden.

Die Salbe der gegenständlichen Erfindung umfasst gemäß einem Aspekt:
- Hauptbestandteil Bienenwachs in Bio-Qualität besonders bevorzugt von westafrikanischen, zertifizierten Imkern, wiederum besonders bevorzugt von Bienenvölkern von Imkern aus Sierra Leone 45% oder von dt. und europäischen Bio Imkern
- Als Emulgator natürliches Bio-Lezithin (je nach Wirkstoffmenge)
- Kakaobutter 30% in Bio-Qualität besonders bevorzugt der Rohstoffkakao aus Madagaskar, Ghana, Sierra Leone, wobei Kakao aus Sierra Leone besonders bevorzugt ist.
- Rest- Öle nach Bedarf, besonders Arganöl und Jojobaöl

Bei den Ölen zeigte sich, dass eine Mischung von 70% Arganöl und 30% Jojobaöl zu bevorzugen ist, da dadurch eine Wirkstofffreigabe und/oder eine Wirkstoffaufnahme optimal ist.

Die Salbe wird gemäß einem Aspekt der vorliegenden Erfindung fertig abgemischt und dann mit der wässrigen Lösung des und/oder der Wirkstoffe versetzt und homogenisiert. Die Lösung enthält 2-5mg des Wirkstoffes bzw. vorzugsweise die gleiche Menge für jeden weiteren erfindungsgemäß verwandten Wirkstoff. Der oder die Wirkstoffe wird oder werden mit Emulgator in doppelt destillierten H2O (5ml bis 10ml) gelöst. Nach Öffnen der Verpackungseinheit ist die Salbe etwa 4 Wochen verwendbar. Sie ist im Kühlschrank bei 5-9 Grad Celsius, besonders bevorzugt bei 7 Grad Celsius aufzubewahren.

Die Einzeldosierung kann zwischen 0,5 und 2 Gramm variieren. Es ist empfehlenswert, die Toxine bzw. den Brennnesselextrakt in eine Salbengrundlage von 25 bis 70 Gramm einzuarbeiten. Die Spinne soll anschließend in die Watte eines sterilen Wattestäbchens beißen, um ihr Gift abzugeben. Alternativ kann auch ein steriler Schwamm verwendet werden. Im Gegensatz zur herkömmlichen elektrischen Melkmethode ermöglicht unsere Vorgehensweise die Gewinnung nativer Gifte.

Die mallorquinische Brennnessel wird als Urtica bianorii bezeichnet. Alle anderen auf Mallorca bekannten Brennnesselarten sind dort nicht endemisch.

Biogene Wirkstoffe, auch als Gesamtgift bezeichnet, umfassen Substanzen, die von Pflanzen, Tieren, Bakterien und Pilzen stammen. Der Begriff Gesamtgift oder Gesamtgiftcocktail beschreibt die Gesamtheit der Substanzen, die aktiv giftige Tiere - also solche, die ihr Gift gezielt zur Beutejagd oder Verteidigung einsetzen - bei diesen Vorgängen abgeben.

Das Gesamtgift wird zur Konservierung auf Temperaturen zwischen -12 und -25 Grad Celsius gefroren. Die Sublimation erfolgt bevorzugt bei 4 Grad Celsius, kann jedoch schonend im Bereich von 3,7 bis 4,3 Grad durchgeführt werden.

Bei Bio-Qualität wird eine Wirkstoffkonzentration von etwa 45 % angestrebt, wobei eine Schwankungsbreite von ±5 % berücksichtigt wird. Generell sind numerische Werte um ±3 % variabel, um eine gewisse Bandbreite abzudecken.

Spinnen werden vor dem Melkvorgang ruhiggestellt, da Stress oder Angst zu Erbrechen führen kann - ähnlich wie beim elektrischen Melken. Dabei kommt es zu molekularen Veränderungen, die die Gewinnung der reinen Substanzen beeinträchtigen.

Die Entnahme erfolgt, indem Rechtshänder die Spinne mit der linken Hand aus dem Behältnis nehmen. Dabei wird die Spinne mit der Bauchseite nach oben gedreht, da das Melken vom Rücken aus nicht möglich ist.

Das Gift wird auf ein steriles Trägermedium übertragen. Nach dem Melkvorgang werden die Spinnen zurück in ihr Behältnis gesetzt.

Die Dauer des Melkens darf pro Spinne fünf Minuten nicht überschreiten. Der Zeitraum umfasst die gesamte Zeitspanne von der Entnahme bis zur Rückführung ins Behältnis.

Die vorliegende Erfindung betrifft eine pharmazeutische Zusammensetzung zur Behandlung erektiler Dysfunktion, die eine pharmazeutisch wirksame Menge an Wirkstoffen enthält, welche aus dem Giftcocktail von Spinnen der Gattung *Phoneutria* gewonnen wurden. Die Zusammensetzung umfasst zudem eine für den jeweiligen Verwendungszweck abgestimmte Menge an Salbengrundlage. Ebenso kann eine pharmazeutische Zusammensetzung bereitgestellt werden, die Wirkstoffe aus dem Giftcocktail von Spinnen der Gattung *Viridasius* oder *Ancylometes* enthält, jeweils in Kombination mit einer geeigneten Salbengrundlage.

Bevorzugt werden für die Gewinnung der Wirkstoffe Arten wie *Phoneutria fera*, *Phoneutria boliviensis*, *Phoneutria eickstedtae* und *Phoneutria pertyi* verwendet. Im Falle der Gattung *Viridasius* wird bevorzugt die Art *Viridasius fasciatus* genutzt, während für die Gattung *Ancylometes* insbesondere die Arten *Ancylometes rufus* und *Ancylometes bogotensis* herangezogen werden. Eine weitere bevorzugte Variante der pharmazeutischen Zusammensetzung zeichnet sich dadurch aus, dass zusätzlich biogene Wirkstoffe verschiedener Gattungen oder Arten kombiniert werden. Ebenso kann die Zusammensetzung alle sieben definierten Wirkstoffe mit den entsprechenden Molekulargewichten der jeweiligen Gattungen bzw. Arten enthalten.

Das Verfahren zur Herstellung dieser pharmazeutischen Zusammensetzung umfasst mehrere Schritte. Zunächst wird der biogene Wirkstoff, also das Gesamtgift der jeweiligen Spinnengattung oder -art, eingeengt und anschließend auf eine Temperatur zwischen minus 12 und minus 25 Grad Celsius gefroren. Danach erfolgt die Sublimation in einem Vakuum bei einer bevorzugten Temperatur von etwa 4 Grad Celsius, wobei ein schonender Prozess auch im Temperaturbereich von 3,7 bis 4,3 Grad Celsius durchgeführt werden kann. Schließlich wird der gewonnene Wirkstoff in eine Salben- oder Cremeträgersubstanz eingearbeitet.

Zur Herstellung der Salbengrundlage werden verschiedene Inhaltsstoffe verwendet. Eine bevorzugte Zusammensetzung enthält Bienenwachs in Bio-Qualität mit einer Konzentration von etwa 45 %, wobei Bienenwachs aus Sierra Leone besonders geschätzt wird. Zusätzlich werden Kakaobutter in Bio-Qualität mit einem Anteil von 30 % sowie verschiedene Öle in einem Gesamtanteil von 20 bis 25 % eingesetzt. Besonders bevorzugt ist eine Mischung aus 70 % Arganöl und 30 % Jojobaöl, da diese Kombination die Aufnahme der Wirkstoffe verbessert.

Die Gewinnung der Wirkstoffe erfolgt durch ein spezifisches Verfahren, bei dem die Spinnen zunächst ruhiggestellt werden. Dies geschieht, indem sie für eine Dauer von 15 bis 30 Minuten in einen Kühlschrank mit einer Temperatur von 10 bis 15 Grad Celsius gestellt werden. Anschließend werden die Spinnen je nach Händigkeit des Anwenders mit der jeweils nicht dominanten Hand aus dem Behältnis entnommen und mit der Bauchseite nach oben gehalten. Das Gift wird entnommen, indem die Spinne bei einer bevorzugten Temperatur von 20 bis 23 Grad Celsius in ein steriles Trägermedium beißt, wobei eine Temperatur von 22 Grad Celsius als besonders optimal gilt. Nach der Entnahme werden die Spinnen zurück in ihr Behältnis gesetzt. Der gesamte Melkvorgang darf pro Spinne eine Dauer von fünf Minuten nicht überschreiten, wobei diese Zeitspanne von der Entnahme bis zur Rückführung in das Behältnis definiert wird.

Die pharmazeutische Zusammensetzung zur Behandlung erektiler Dysfunktion bietet mehrere Vorteile, die sich aus der gezielten Auswahl biogener Wirkstoffe und der speziellen Herstellungsweise ergeben. Ein zentraler Aspekt ist die Verwendung von Wirkstoffen aus den Giftcocktails der Spinnen-Gattungen *Phoneutria*, *Viridasius* und *Ancylometes*. Diese enthalten natürliche bioaktive Substanzen, die nachweislich die Durchblutung fördern und eine muskelentspannende Wirkung haben. Dadurch kann die pharmazeutische Zusammensetzung eine vielversprechende Alternative zu synthetischen PDE-5-Hemmern wie Sildenafil darstellen. Zudem ist durch die lokale Anwendung eine gezielte Wirkung möglich, während systemische Nebenwirkungen, die bei oraler Einnahme auftreten könnten, minimiert werden.

Ein weiterer Vorteil liegt in der individuell abgestimmten Menge an Salbengrundlage. Diese ermöglicht eine flexible Dosierung und erleichtert die Anwendung, da die Wirkstoffe direkt an der betroffenen Stelle aufgenommen werden. Die topische Applikation sorgt für eine schnelle Wirkung und reduziert das Risiko unerwünschter Wechselwirkungen mit anderen Medikamenten.

Besonders innovativ ist zudem die Kombination biogener Wirkstoffe aus verschiedenen Gattungen und Arten. Durch diesen Ansatz können synergistische Effekte erzielt werden, wodurch sich die Effektivität der Behandlung steigert. Je nach Bedarf kann die Formulierung individuell angepasst werden, um eine optimale Wirkung zu erzielen. Dies ist insbesondere für Patienten von Vorteil, die unterschiedlich auf bestimmte Wirkstoffe reagieren.

Auch das Herstellungsverfahren trägt erheblich zur Qualität der pharmazeutischen Zusammensetzung bei. Die Gewinnung der Wirkstoffe erfolgt durch ein Verfahren, bei dem die Spinnen zunächst bei einer Temperatur von 10 bis 15 Grad Celsius ruhiggestellt werden. Dies verhindert Stressreaktionen wie Erbrechen, die zu molekularen Veränderungen führen und die Reinheit der Wirkstoffe beeinträchtigen könnten. Anschließend erfolgt die Entnahme des Giftes durch einen natürlichen Beißvorgang in ein steriles Trägermedium, wobei die Spinnen bei einer optimalen Temperatur von etwa 22 Grad Celsius gehalten werden. Dadurch bleibt die chemische Integrität der Toxine erhalten, im Gegensatz zu herkömmlichen elektrischen Melkverfahren, bei denen die Struktur der Wirkstoffe verändert werden kann.

Ein weiterer Vorteil ergibt sich aus der gezielten Konservierung des Gesamtgiftes. Durch Einengen und Einfrieren auf eine Temperatur zwischen minus 12 und minus 25 Grad Celsius wird die Haltbarkeit der Wirkstoffe verlängert. Die anschließende Sublimation unter Vakuum bei etwa 4 Grad Celsius sorgt für eine besonders schonende Aufbereitung, sodass die bioaktiven Komponenten in ihrer ursprünglichen Form erhalten bleiben.

Auch die Auswahl der Salbengrundlage trägt zur Effektivität des Produkts bei. Die Kombination aus Bienenwachs, Kakaobutter und hochwertigen Ölen in Bio-Qualität stellt sicher, dass die Wirkstoffe optimal in die Haut eindringen. Besonders die Mischung aus 70 % Arganöl und 30 % Jojobaöl verbessert die Aufnahmefähigkeit und sorgt für eine langanhaltende Wirkung. Dabei wird Bienenwachs aus Sierra Leone bevorzugt, da es eine besonders hohe Qualität aufweist.

Zusammenfassend bietet die pharmazeutische Zusammensetzung zahlreiche Vorteile durch die Verwendung natürlicher Wirkstoffe, die gezielte Applikation, die Kombination verschiedener biogener Substanzen und ein schonendes Herstellungsverfahren. Dies macht sie zu einer innovativen und vielversprechenden Alternative zu herkömmlichen Behandlungsansätzen bei erektiler Dysfunktion.

Die Wirkstoffe der pharmazeutischen Zusammensetzung werden gezielt über ihr Molekulargewicht spezifiziert, da diese Angabe eine präzise Identifikation, Standardisierung und Reproduzierbarkeit der Formulierung ermöglicht. Jeder Wirkstoff innerhalb des Giftcocktails einer bestimmten Spinnengattung oder einer Pflanze besteht aus einer Vielzahl unterschiedlicher bioaktiver Peptide und Proteine. Da die Zusammensetzung des Gifts selbst innerhalb einer Art variieren kann, bietet die Festlegung eines spezifischen Molekulargewichts eine eindeutige Definition der Substanzen, die für die gewünschte pharmakologische Wirkung verantwortlich sind.

Durch diese Vorgehensweise wird sichergestellt, dass die pharmazeutische Zusammensetzung in jeder Produktion eine gleichbleibende Qualität und Wirksamkeit aufweist. Da selbst innerhalb einer Spinnenpopulation natürliche Schwankungen auftreten können, ist die Festlegung des Molekulargewichts als Maßstab essenziell, um unerwünschte Variationen zu vermeiden und eine standardisierte Wirkstoffkonzentration zu gewährleisten.

Darüber hinaus ist das Molekulargewicht ein entscheidender Faktor für die pharmakologische Aktivität der Wirkstoffe. Es beeinflusst maßgeblich deren Aufnahme, Verteilung und Wirksamkeit im Körper. Kleinere Peptide können beispielsweise schneller absorbiert und verstoffwechselt werden, während größere Moleküle eine längere Halbwertszeit aufweisen und eine nachhaltigere Wirkung entfalten können. Indem gezielt Wirkstoffe mit bestimmten Molekulargewichten ausgewählt werden, lässt sich die gewünschte therapeutische Wirkung optimieren und gleichzeitig unerwünschte Nebenwirkungen minimieren.

Zusätzlich ermöglicht diese Art der Spezifikation eine verbesserte analytische Kontrolle während des Herstellungsprozesses. Mittels Verfahren wie der Massenspektrometrie oder der Gel-Elektrophorese kann sichergestellt werden, dass genau die definierten bioaktiven Substanzen in der pharmazeutischen Zusammensetzung enthalten sind. Dies trägt wesentlich zur Qualitätssicherung bei und erleichtert regulatorische Zulassungsprozesse, da die Wirkstoffe objektiv und reproduzierbar nachgewiesen werden können.

Zusammenfassend dient die Spezifikation der Wirkstoffe über ihr Molekulargewicht dazu, eine hohe pharmazeutische Qualität, präzise Dosierung und optimale Wirksamkeit der Zusammensetzung sicherzustellen. Dies ermöglicht eine gezielte therapeutische Anwendung, die den Anforderungen einer standardisierten und wirksamen Behandlung gerecht wird.

Eine besonders bevorzugte Kombination von Wirkstoffen für die Behandlung erektiler Dysfunktion umfasst Peptide aus den Giften von *Phoneutria boliviensis* (16,2 kDa), *Phoneutria eickstedtae* (15,4 kDa), *Ancylometes rufus* (27,6 kDa) und *Viridasius cf. fasciatus* ("forestform", 63,2 kDa). Diese Zusammenstellung bietet eine optimierte Wirkung auf die Gefäßerweiterung, da sie gezielt Kalium- und Kalziumkanäle moduliert, was zu einer verbesserten Durchblutung im Schwellkörper führt. Insbesondere die Toxine aus *Phoneutria*-Arten sind für ihre starke gefäßerweiternde Wirkung bekannt, während *Ancylometes rufus* durch seine langanhaltende Modulation der glatten Muskulatur den Effekt verstärkt. *Viridasius cf. fasciatus* trägt zudem durch eine verstärkte Stickstoffmonoxid-Freisetzung zur anhaltenden Entspannung der Blutgefäße bei.

Diese Kombination eignet sich besonders für die Herstellung einer topischen Salbe, die direkt auf die Haut aufgetragen wird, um eine schnelle Wirkung zu erzielen. Insbesondere Patienten mit leichter bis mittlerer erektiler Dysfunktion profitieren von dieser Mischung, da sie nicht nur die Durchblutung verbessert, sondern auch die Dauer der Wirkung verlängert.

Eine weitere bevorzugte Formulierung umfasst *Phoneutria fera* (13,6 kDa), *Phoneutria cf. nigriventer* (5,8 kDa) und *Urtica bianorii* (15,3 kDa). Diese Kombination bietet nicht nur gefäßerweiternde Effekte, sondern hat auch entzündungshemmende Eigenschaften, was sie besonders für Patienten mit vaskulär bedingter erektiler Dysfunktion oder begleitenden Entzündungsprozessen interessant macht. Die Inhaltsstoffe der mallorquinischen Brennnessel (*Urtica bianorii*) wirken als natürlicher Modulator auf das Stickstoffmonoxid-System, was die Wirkung der Spinnengifte ergänzt und für eine gleichmäßige Vasodilatation sorgt.

Für eine maximale Wirkungsdauer kann die Kombination von *Phoneutria pertyi* (15,2 kDa), *Ancylometes bogotensis* (28,2 kDa) und *Viridasius cf. fasciatus* (63,2 kDa) verwendet werden. Diese Wirkstoffmischung ermöglicht eine langanhaltende Wirkung durch gezielte Hemmung der Phosphodiesterase-5 (PDE-5), die für den Abbau von cGMP in den Schwellkörpern verantwortlich ist. Durch die Blockade von PDE-5 bleibt der Blutfluss länger erhöht, was zu einer verlängerten Erektionsfähigkeit führt. Diese Zusammensetzung ist besonders für Patienten mit chronischer erektiler Dysfunktion geeignet, die eine Alternative zu herkömmlichen PDE-5-Hemmern suchen.

Die gezielte Auswahl von Wirkstoffen mit spezifischen Molekulargewichten stellt sicher, dass jede Formulierung eine konstante und vorhersehbare Wirkung aufweist. Die Wirkstoffe können je nach gewünschter Wirkung kombiniert werden, um entweder eine schnelle, langanhaltende oder synergistische Gefäßerweiterung zu erzielen. Diese Flexibilität ermöglicht eine individuell abgestimmte Therapie, die den Bedürfnissen unterschiedlicher Patienten gerecht wird.

Bio-Qualität in der pharmazeutischen Zusammensetzung bedeutet, dass die verwendeten Rohstoffe aus nachhaltigem, kontrolliert biologischem Anbau oder aus zertifizierten natürlichen Quellen stammen. Dabei wird sichergestellt, dass keine synthetischen Pestizide, Herbizide oder chemischen Düngemittel eingesetzt werden, um eine möglichst reine und naturbelassene Qualität der Inhaltsstoffe zu gewährleisten.

Bei pflanzlichen Komponenten, wie den verwendeten Ölen und Wachsen, bedeutet Bio-Qualität, dass sie ohne den Einsatz von genmanipulierten Organismen (GMO-frei) produziert und geerntet werden. So stammt das in der pharmazeutischen Zusammensetzung verwendete Bienenwachs aus nachhaltiger Imkerei, in der die Bienen artgerecht gehalten werden und keine chemischen Zusätze oder antibiotische Behandlungen im Bienenstock erfolgen. Besonders bevorzugt ist Bienenwachs aus Sierra Leone, da es für seine hohe Reinheit und seine besondere Zusammensetzung an natürlichen Estern und Fettsäuren bekannt ist, die die Hautverträglichkeit der Salbengrundlage verbessern.

Auch die verwendeten Öle, darunter Arganöl und Jojobaöl, entsprechen hohen Bio-Standards. Sie werden durch mechanische Pressung ohne chemische Lösungsmittel gewonnen, um eine maximale Reinheit zu gewährleisten. Zudem stammen sie aus kontrolliert biologischem Anbau, was bedeutet, dass sie ohne synthetische Zusatzstoffe hergestellt wurden und ihre natürlichen Wirkstoffe vollständig erhalten bleiben. Die bevorzugte Mischung aus 70 % Arganöl und 30 % Jojobaöl sorgt nicht nur für eine verbesserte Aufnahme der Wirkstoffe in die Haut, sondern unterstützt gleichzeitig die Zellregeneration und Feuchtigkeitsversorgung.

Die Kakaobutter in der pharmazeutischen Zusammensetzung stammt ebenfalls aus biologischem Anbau und wird durch schonende Verfahren extrahiert, um ihre pflegenden Eigenschaften zu bewahren. Da sie reich an natürlichen Antioxidantien und Fettsäuren ist, verbessert sie die Hautdurchlässigkeit der aktiven Wirkstoffe und trägt zur Stabilität der Salbengrundlage bei.

Insgesamt bedeutet Bio-Qualität in dieser pharmazeutischen Zusammensetzung nicht nur den Verzicht auf schädliche Chemikalien in der Herstellung, sondern auch eine nachhaltige Gewinnung und Verarbeitung der Rohstoffe. Dies stellt sicher, dass die pharmazeutische Salbe nicht nur eine hohe Wirksamkeit aufweist, sondern auch hautfreundlich und umweltverträglich ist. Patientenbeispiele:
- Patient heute 37 Jahre alt, hat seit der Pubertät an eDys (erektile Dysfunktion) gelitten. Mit 21 hat er erstmal die Salbe erhalten und erstmals eine Erektion nach der 5. oder 6. Anwendung bekommen. Patient musste die Salbe nach eigenen Aussagen etwa 5 Jahre lang mit einer 1,5 jährigen Pause (keine Partnerin gehabt) nehmen. Seit 1999 braucht er die Salbe nicht mehr und berichtet, dass er selbständig eine Erektion bei genügend Erregungszustand bekommen kann.
- Patient heut 55 Jahre alt, nach Trennung von seiner Frau und seinen Kindern, war er trotz medizinischer Behandlung nicht mehr in der Lage eine Erektion zu bekommen. Schon bei der zweiten Verwendung der Salbe 1992 bekam er mit seiner neuen Lebensgefährtin und jetzigen Frau wieder eine Erektion. Die Salbe konnte er nach 3 Jahren wieder absetzen und hat nach eigenen Aussagen keine "Probleme mehr beim Sex".
- Bei einem Patienten der nie eine Erektion hatte wurde eine erektile Dysfunktion diagnostiziert. Heute 40 Jahre alt. Mit 19 Jahren kennengelernt und teilweise auch über seine Probleme gesprochen. Wollte auch, dass ihm eine Salbe gemischt wurde. Schon bei der ersten, alleinigen Anwendung bekam er eine Erektion. Muss die Salbe (evtl. als psychisch/psychologische Hilfestellung) bis heute nehmen, bekommt aber bei 3 von 5 Versuchen bzw. Anwendungen eine Erektion.
- Patient heute 44 Jahre alt leidet - nach eigenen Aussagen- schon immer an erektiler Dysfunktion, die ihm mit 20 auch diagnostiziert wurde. Hat die Salbe - aufgrund der Bekanntschaft und aufgrund Vertrauens- 5 Monate angewendet und keine Erektion bekommen, auch nicht bei Wirkstofferhöhung. Nachdem der Wirkstoff verändert wurde (neben Spinnen der Gattung Phoneutria auch von Spinnen der Gattung Ancylometes) - 3 Wirkstoffe in der Salbebekam er nach ca. 20 Anwendungen (Patient weiß das nicht mehr genau) seine erste Erektion auch gleich mit Ejakulation. Dieser Patient nimmt die Salbe laut eigener Aussage "sicherheitshalber" bis heute, wenn er "mit seiner Frau schläft".
- Patient heute 38 Jahre alt, hörte von seinem Bekannten von der Salbe (Bekannter ist erwähnter Patient mit 55 Jahren) und wurde samt Arztbericht, in dem zu lesen war, dass er an erektiler Dysfunktion leide, vorstellig. Er berichtet bei einem Treffen, ca. 3 Monate nachdem er mit der Behandlung angefangen hatte, dass bei ihm die Einreibung an der Peniswurzel (weder durch ihn selbst, noch durch seine Freundin) zu einem "Erfolg geführt" hätte. Erst als seine Freundin auf die Idee kam die Salbe unter die Vorhaut, direkt in die Eichel einzumassieren, bekam der Mann wirklich eine Erektion. Auch dieser Patient braucht die Salbe bis heute.

## Patentansprüche

1. Pharmazeutische Zusammensetzung zur Behandlung einer erektilen Dysfunktion mit einer pharmazeutisch wirksamen Menge von mindestens einem Wirkstoff, die Menge aufweisend:
| | |
|---|---|
| Phoneutria fera | 13,6 kDA; |
| Phoneutria depilata | 14,1 kDA; |
| Phoneutria boliviensis | 16,2 kDA; |
| Phoneutria eickstedtae | 15,4 kDA; |
| Phoneutria pertyi | 15,2 kDA; |
| Phoneutria cf. nigriventer | 5,8 kDA; |
| Urtica bianorii | 15,3 kDA |
| Viridasius cf. fasciatus "forestform" | 63,2 kDA; |
| Ancylometes bogotensis | 28,2 kDA; und/ oder |
| Ancylometes rufus | 27,6 kDA, |
wobei zuerst eine den Wirkstoff bereitstellende Spinnengattung angegeben ist und danach ein spezifisches Molekulargewicht des jeweiligen Wirkstoffes.

2. Zusammensetzung nach Anspruch 1, wobei die Menge in eine Salbengrundlage eingebracht ist.

3. Zusammensetzung nach Anspruch 1 oder 2 aufweisend mindestens einen Zusatzstoff mit Öl, Kakaobutter und/ oder Bienenwachs.

4. Zusammensetzung nach Anspruch 3, wobei der mindestens eine Zusatzstoff in Bio-Qualität vorliegt.

5. Zusammensetzung nach Anspruch 3 oder 4, wobei das Öl Arganöl und/ oder Jojobaöl aufweist.

6. Zusammensetzung nach Anspruch 3 bis 5, wobei das Öl 70 % Aranöl und 30 % Jojobaöl aufweist.

7. Zusammensetzung nach einem der vorhergehenden Ansprüche, wobei der mindestens eine Wirkstoff in einer Einzeldosierung von 0,5 bis 2 Gramm vorliegt.

8. Zusammensetzung nach einem der vorhergehenden Ansprüche, aufweisend Mallorquinische Brennnessel.

9. Verfahren zur Herstellung einer pharmazeutischen Zusammensetzung nach einem der Ansprüche 1 bis 8, **dadurch gekennzeichnet**, aufweisend:
- ein Ruhigstellen von Spenderspinnen bei einer Temperatur von 10 bis 15 Grad Celsius; und
- ein Melken der Spinnen bei einer Temperatur von 20 bis 23 Grad Celsius mittels mindestens einem sterilen Beisblock.

10. Verfahren nach Anspruch 9, wobei die mindestens eine Spenderspinne 15-30 Minuten ruhig gestellt wird.

11. Verfahren nach Anspruch 9 oder 10, wobei das Melken bei einer Temperatur von 22 Grad Celsius erfolgt.

12. Verfahren nach einem der Ansprüche 9 bis 11, **dadurch gekennzeichnet, dass** der mindestens eine Wirkstoff auf minus 20 Grad gefroren wird und in ein Vakuum verbracht und auf + 4 Grad Celsius sublimiert wird.

13. Verfahren nach einem der Ansprüche 9 bis 12, wobei der mindestens eine Wirkstoff in eine Salben- und/ oder Cremeträgersubstanz eingebracht wird.

14. Verfahren nach einem der Ansprüche 9 bis 13, wobei der der mindestens eine Wirkstoff über einen Emulgator in 5-10ml doppelt destilliertes H2O eingearbeitet wird.
